Europäisches Patentamt

European Patent Office

Office européen des brevets

(11) Publication number: **0 366 438**
**A1**

# EUROPEAN PATENT APPLICATION

(21) Application number: 89310989.2

(51) Int. Cl.⁵ **C12P 19/34 , C12N 15/10**

(22) Date of filing: 25.10.89

(30) Priority: 25.10.88 IE 3226/88

(43) Date of publication of application:
02.05.90 Bulletin 90/18

(34) Designated Contracting States:
**AT BE CH DE ES FR GB GR IT LI LU NL SE**

(71) Applicant: **DNA PREP GALWAY LIMITED**
**University College Galway**
**Galway(IE)**

(72) Inventor: **Gannon, Bernard Francis Xavier**
**"Murgan" 12 Pollnarooma**
**Salt Hill Galway(IE)**
Inventor: **Fotsis, Theodore**
**Heidelbergerstrasse 54b**
**D-6903 Neckargemund-3(DE)**
Inventor: **Murphy, Carol Elizabeth**
**Heidelbergerstrasse 54b**
**D-6903 Neckargemund-3(DE)**

(74) Representative: **Goldin, Douglas Michael et al**
**J.A. KEMP & CO. 14, South Square Gray's Inn**
**London WC1R 5EU(GB)**

(54) Method for the separation of nucleic acids from protein material based on cation exchangers.

(57) A method for the separation of nucleic acid from protein which method comprises contacting a solution containing a mixture of nucleic acid and protein with a cation exchanger at a pH below the isoelectric point of the protein and recovering said nucleic acid. If necessary, the mixture may be incubated with a proteinase prior to its application to the cation exchanger. In addition sodium dodecyl sulphate may also be used with the proteinase. The recovered nucleic acid is suitable for DNA probe analysis.

## METHOD FOR THE SEPARATION OF NUCLEIC ACIDS FROM PROTEIN MATERIAL BASED ON CATION EXCHANGERS

This invention relates to a method for the separation of nucleic acids from protein material based on cation exchangers.

One of the most common procedures in Molecular Biology is manipulation of nucleic acids such as DNA with restriction or modifying enzymes. After such a manipulation. the successful performance of subsequent steps, in many cases, depends on the complete removal of the used enzyme. Phenol extraction is the most commonly used method for removal of enzymes from nucleic acid solutions. A typical example of a common and procedure in molecular cloning is dephosphorylation of nucleic acids by alkaline phosphatase (Maniatis, T., Fritsch, E.T., and Sambrook, 3. (1982) "Molecular cloning: A Laboratory Manual", Cold Spring Harbour Laboratory, Cold Spring Harbour, New York). However, successful subsequent ligation of the dephosphorylated nucleic acid to another nucleic acid molecule requires complete removal of alkaline phosphatase. This is currently achieved by successive phenol/chloroform extractions followed by ethanol precipitation (Maniatis, T., Fritsch, E.T., and Sambrook 3. (1982) op cit.).

In order to isolate DNA from a given biological material (blood, human, animal and plant tissues, bacterial and cell cultures, etc.) it is necessary to separate it from proteins. Extraction of genomic DNA im poses further difficulties as it is closely bound to histones and other proteins in situ. Traditionally, the separation of DNA from proteins and other impurities has involved a combination of the use of proteinases and organic solvents, such as phenol. Removal of the undesirable traces of organic solvents is then frequently achieved with a time consuming dialysis step. DNA in purified form is currently required for many applications. Also a considerable body of data points to a major role for DNA probes in the future of the diagnostics industry. However, current DNA extraction methods are a limiting factor towards the widespread use of DNA probes in the diagnostics field. Therefore, any method which would accelerate and simplify DNA preparation would be of particular importance in many applications involving DNA, especially if it is amenable for automation.

Phenol extraction is the most commonly used method for deproteinization of nucleic acid solutions. However, there are several drawbacks regarding its use. Phenol is a toxic organic solvent and as a strong protein denaturating agent can cause serious burns on contact with skin which often require emergency treatment. Though many suppliers now provide clear and colourless liquified phenol which can be used directly without purification the less expensive crystalline form requires redistillation prior to use. Redistillation is also required when stored phenol acquires a yellow or pink coloration. The coloration is indicative of the presence of phenolic oxidation products (quinones, diacids, etc.) which form during storage. These contaminants must be removed because they cause cleavage of phosphodiester bonds and cross-linking of DNA strands. Indeed, phenol extraction is known to cause nicks in the DNA. Redistillation of the toxic phenol is time consuming and potentially dangerous. It should be carried out in a fume hood with special care and the volume of phenol in the distillation flask should never be allowed to drop below 100 ml, as the concentrated residue is explosive. Phenol should be saturated by equilibration with the relevant extraction buffer prior to use. The need for a shaking and a separation step is particularly undesirable for manual or automated systems. Much of the protein segregates to the white flocculent interphase and as a result part of the upper aqueous phase (containing the DNA) should be left behind leading to losses of nucleic acids.

Soviet Union patent Application No. SU-A-1373709 (Paponov et al) discloses a method for separating desoxyribonucleic acids from desoxyribonucleoproteins using salt precipitation followed by selection of the DNA by a strongly acidic cation exchange resin. The cation exchange resin in question is not in the form of DNA deproteinising agent, but is an adsorbent competing with DNA for proteins in the course of the reduction of the salt concentration by dialysis. In this method DNA solutions are first deproteinized using high salt concentrations and then contacted with the cation exchange resin, the ionic strength of the solution being gradually reduced by means of simultaneous dialysis. However, this method involves a number of steps and takes several hours to complete. Furthermore, it would not be suitable for small scale, rapid preparation of genomic DNA and rapid removal of modifying enzymes and restriction endonucleases from DNA. The quality of the DNA separated by the method of SU-A-1373709 is unknown since no data, such as digestability by restriction endonucleases, absorption spectrum or suitability of the purified DNA for use as a target for DNA probes, is given. It is likely that many proteins would bind to a cation exchanger under the pH conditions employed in SU-A-1373709. Finally, completely ignoring the practical aspects mentioned above, it is not clear that the method in SU-A-1373709 could be applied to the precipitation of DNA from microorganisms without many modifications as the use of detergents or proteolytic enzymes could interfere with the absorption of the denatured protein onto the cation exchanger.

It is an object of the present invention to provide an accelerated and simplified procedure for nucleic acid sample preparation which obviates the use of toxic organic solvents, high salts or dialysis as discussed above.

Accordingly, the invention provides a method for the separation of nucleic acid from protein, which method comprises contacting a solution containing a mixture of nucleic acid and protein with a cation exchanger at a pH below the isoelectric point of the protein and recovering said nucleic acid.

At sufficiently low pH (below the isoelectric point) proteins are positively charged and thus retained on cation exchangers. On the contrary, nucleic acids are repelled from the cation exchangers because of the negative charge of the phosphate functions. As there is no interaction between nucleic acids and cation exchangers, the recovery of the former can be quantitative provided that inert matrices are used. The protein may, if desired, be recovered by raising the pH of the cation exchanger so as to remove the protein therefrom which may be eluted and collected by well-known techniques.

It is customary to increase the salt concentration at this step also to aid in the dissociation of the proteins from the cation exchanger. However, the nucleic acid solution is recovered prior to that step thereby providing a solution of nucleic acid in a buffer which can be used for subsequent steps without dialysis.

Accordingly, it will be appreciated that the method according to the invention has immediate applicability to research procedures which involve manipulations of DNA or RNA and which currently require phenol extractions.

According to one embodiment of the invention, the solution of nucleic acid and protein is applied to a column of a suitable cation exchanger and the nucleic acid is eluted from the column and collected. However, the method according to the invention may also be carried out using a cartridge, batch or membrane system.

Prior to contacting the solution of nucleic acid and protein with the cation exchanger, the mixture of nucleic acid and protein may be preincubated with a proteinase and, if necessary sodium dodecyl sulphate (SDS). However, other procedures including the use of acids, alkali, volatile chaotropic salts or other conventional methods, followed by neutralization may also be used. Such a preincubation step would normally be required in the preparation of tissue or the like samples for DNA or RNA probe analysis which is a useful extension of the method according to the invention.

Unlike the liquid-liquid phenol extraction or salt precipitation and dialysis techniques used heretofore, the solid-liquid cation exchange system in accordance with the method of the invention can be readily incorporated into instruments for automated DNA or RNA preparation from biological or other materials.

The cation exchanger may be a weak cation exchanger or a strong cation exchanger depending on the separation required, as demonstrated hereinbelow in the Examples. The solution is suitably a low molarity buffer. The pH of the solution will normally be in the range 3.5-6.5.

The invention also provides a cation exchanger for use in a method as hereinabove specified.

Generally, and in accordance with one embodiment of the invention, the sample to be separated is loaded onto a preequilibrated column of the cation exchanger in a low molarity buffer pH of approximately 6.0. In such cases, the DNA, which is negatively charged at pH ~6.0, is eluted in the loading buffer whereas proteins are retained and eluted only after the change of pH, for example washing with a buffer at a pH of approximately 9.0. This separation appears to be absolute, particularly as judged by the very sensitive assay for the presence of alkaline phosphatase as used in Example 1 below.

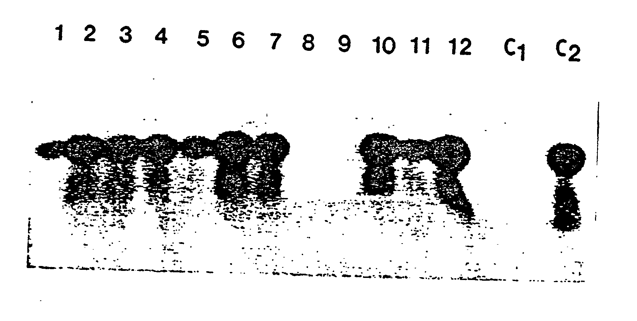

The accompanying Figure is an autoradiogram of PAGE (polyacrylamide gel electrophoresis) analysis of the fractions obtained from cation exchange chromotography of alkaline phosphatase described in Example 1

The invention will be further illustrated by the following Examples.

## EXAMPLE 1

Alkaline phosphatase was used as a model to demonstrate the method of the invention. To this end a microgranular carboxymethylcellulose weak cation exchanger (CM 52, Whatman) was utilized. The cation exchanger was packed in a silanized Pasteur pipette to give a bed volume of 1 ml. Two columns were prepared. To the first column 500 ng of lamda DNA digest with Hind III was applied in 100 microlitres of 10 mmole/l ammonium acetate buffer at pH 6.0. To the second column 100 U of calf intestine alkaline phosphatase (EC 3.1.3.1) obtained from Boehringer Mannheim was applied also in 100 microlitres of 10 mmole/l ammonium acetate buffer at pH 6.0. Each column was further eluted with 100 microlitres of the same buffer and the eluents, which included the application volume, were collected in one fraction (f₁). After that three more fractions of 200 microlitres of the ammonium acetate buffer were collected (f₂₋₄). Finally, the columns were eluted with eight fractions of 200 microlitres of 50 mmole/l Tris-HC1, 0.1 mmole/l EDTA, 300 mmole/l NaCl, pH 9.0 (f₅₋₁₂). The elution pattern of nucleic acids was established by agarose gel electrophoresis of the fractions obtained from the first column. The elution pattern of alkaline phosphatase was established by detecting its activity in the following way. To 20 microlitres of each fraction eluted from the second column 10 microlitres of a 5'-end labelled synthetic 30 mer (2.5 x 10⁵ cpm) and 170 microlitres of alkaline phosphatase reaction buffer. (50 mmole/l Tris-HCl, 0.1 mmole EDTA, pH 8.0) were added. The samples were incubated at 50°C for 60 min. after which they were subjected to polyacrylamide gel electrophoresis under denaturing conditions. The gel was autoradiographed and the alkaline phosphatase activity was detected by the lack of the oligonucleotide band in the corresponding fractions as a result of dephosphorylation. The results are depicted in the accompanying Figure. In relation to the Figure. C₁ is a control to which alkaline phosphatase, labelled synthetic oligonucleotide and alkaline phosphatase reaction buffer were added whereas C₂ is a control which contained only labelled oligonucleotide and alkaline phosphatase reaction buffer. From the autoradiogram it is clearly evident that when alkaline phosphatase is present (C₁, 8, 9) the ammonium acetate oligonucleotide band is absent due to dephosphorylation and increased mobility of the released labelled phosphate. Nucleic acids were eluted mainly in f₃, while alkaline phosphatase was retained on the column, being eluted by increasing the pH and salt concentration of the elution solvent (f₈₋₉). The recovery of the lamda Hind III fragments was quantitative.

## EXAMPLE 2

The procedure of Example 1 was generally followed using S Sepharose Fast Flow (Pharmacia, Sweden) strong cation exchanger. The column was successively washed with 10 bed volumes of 0.2 mole/l HCl, distilled water, 0.2 mole/l ammonium acetate, pH 4.8 (hereinafter AA, pH 4.8) and 10 mmole/l AA, pH 4.8. Large batches of cation exchanger can be stored in the latter solvent in 20% ethanol at 4°C. The elution system was that used in Example 1; the stronger nature of the Sepharose exchanger allowed the use of lower starting pH (4.8). Nucleic acids representative of the entire size and configuration (i.e. linear, supercoiled, relaxed circular) spectrum were chromatographed through the cation exchanger and an excellent recovery was obtained in each case. Furthermore, the nucleic acids could be promptly digested, ligated and cloned. A number of proteins, namely albumin and globulin, restriction enzymes, for example. Hind III and other enzymes used in molecular biology, namely pronase, were also chromatographed and none of them was eluted with AA, pH 4.8; they all required higher pH and salt concentration for their elution. Therefore, a clear separation of nucleic acids and proteins was observed.

For DNA binding enzymes, including restriction enzymes, (but not including alkaline phosphate) treatment with a detergent such as sodium dodecyl sulphate was necessary to dissociate the protein from the DNA. In an alternative use of the invention the cation exchanger could be used to distinguish between DNA binding proteins (many of which are of biological importance and those that are not tightly associated with DNA).

It will be appreciated that proteolytic enzymes other than Proteinase K and detergents other than SDS can be incorporated into the procedure without altering the generality of the method. The use of other detergents might be particularly beneficial as they would allow omission of an ethanol precipitation step.

As indicated above, the DNA recovered in Examples 1 and 2 can be readily digested by restriction enzymes or ligated. There does not appear to be a size effect limitation; both synthetic oligonucleotides of 30 bases and genomic DNA were recovered in good yield.

As indicated, Examples 1 and 2 show that for all of the proteins tested, under appropriate conditions of pH, DNA does not bind to the cation exchanger whereas proteins do. When the DNA and proteins were present in a sample placed on a column or treated in a batch manner similar results were obtained in many cases. When restriction enzymes (e.g. Hind III) are used, prior addition of SDS was needed to dissociate the DNA from the DNA binding protein as indicated above. Based on these observations. it was postulated that DNA of biological origin (rather than the prepurified DNA used in Examples 1 and 2) could also be purified by the method according to the invention which could include a cation exchanger.

## EXAMPLE 3

The method according to the invention has been applied to the preparation of genomic DNA from crude biological samples including microorganisms and eucaryote or eucaryotic tissues. A food sample such as yoghurt, chicken samples etc. or biological sample such as blood, semen etc. may be used. In one example of this, 1.5 ml of an overnight culture of E. coli was centrifuged in a bench top Eppendorf type microfuge at approximately 10000g for 1 minute. The supernatant was discarded and the pellet resuspended in 500 microlitres Tris-HCl 10 mM pH 8.0, NaCl 40mM, EDTA, 2mM. Proteinase K and SDS were added to final concentrations of 200 micrograms/ml and 0.5% respectively. The pH was subsequently reduced to approximately pH 4.8 by the addition of 1 mol/l ammonium acetate (A.A.) pH 4.8 solution to a final concentration of 25 mmol/l. Following pH adjustment, 500 microlitres of a 1:1 slurry of the ammonium form of the cation exchanger S-Sephadex fast flow (Pharmacia) in 10 mmol/l of A.A. pH 4.8 were added. The sample was agitated gently for periods up to 30 minutes. The tube was then placed in a rack to allow the cation beads to settle. The supernatant was removed and transferred to a new tube and DNA recovered and concentrated by ethanol precipitation. The yield of DNA was comparable with results obtained by conventional techniques.

The method was highly repeatable with consistent yields of DNA which was readily digested by restriction enzymes. DNA prepared in this way has also been used successfully as a target for DNA probes.

To indicate the generality of the method, similar experiments were performed on a tissue of biological origin which would be difficult to process using phenol extraction based methods.

EXAMPLE 4

Fish fins, on which DNA probe analysis was required, were added to appropriate solutions of Proteinase K and SDS so as to immediately dissolve the tissue. The proteins and protein fragments were released together with the DNA and RNA. The pH of the solution was changed to below the isoelectric point (about pH 4.8). The cation exchange resin was added as previously described and a sample collected from the eluate for subsequent probe analysis.

The conditions used in the above Examples have been found to be quite effective. However, conditions can be further tailored to meet specific deproteinization needs. Also it will be appreciated a variety of cation exchangers can be used in addition to the two types of cation exchangers used in the Examples. Therefore, in accordance with the invention ready-to-use packed cation exchange column prepared in the manner described above offers a very efficient alternative for the separation of DNA from proteins to laboratories involved in molecular biology. The method in accordance with the invention avoids distillation and equilibration of toxic phenol, it is clean, rapid and quantitative, it does not affect the DNA and it does not impose any considerable dilution factor. These advantages will be readily appreciated by those skilled in the art.

A useful extension of the method according to the invention is in the preparation of DNA samples for DNA probe analysis.

The method according to the invention has commercial uses which include, but are not limited to the following:-

1. The preparation of DNA from microorganisms for identification using DNA probes.

2. The preparation of DNA from human samples for genetic analysis using DNA probes.

3. The treatment of therapeutic proteins (e.g. interferon, interleukin) expressed in animal cells to remove any contaminating DNA (provided the protein was stable under the pH conditions chosen).

4. In molecular biology research laboratories where DNA has frequently to be separated from modifying enzymes at the end of a manipulation.

5. In plasmid preparation steps for molecular biology research laboratories.

The formats that can be used depend on the use envisaged but include:

(a) A prepacked cation exchange cartridge which may be fitted to a syringe which contained the sample at the correct pH. In this case only the DNA would be recovered (uses 1, 2, 4 and 5 above).

(b) A prepacked column for larger scale processes (uses 1, 2, 4 and 5 above). By a change of the buffer conditions the proteins required could be recovered from the exchanger (use 3 above).

(c) A cation exchange membrane. This could be used in situations similar to those described for (a) and (b) above.

(d) Batch-wise treatment of samples with a cation exchanger.

**Claims**

1. A method for the separation of nucleic acid from protein, which method comprises contacting a solution containing a mixture of nucleic acid and protein with a cation exchanger at a pH below the isoelectric point of the protein and recovering said nucleic acid.

2. A method according to Claim 1, wherein the solution of nucleic acid and protein is applied to a column of a suitable cation exchanger and the nucleic acid is eluted from the column.

3. A method according to Claim 1, which is carried out using a cartridge, batch or membrane system.

4. A method according to any preceding claim, wherein the mixture of nucleic acid and protein is incubated with a proteinase prior to application of the mixture to the cation exchanger.

5. A method according to Claim 4 wherein the mixture is also incubated with sodium dodecyl sulphate.

6. A method according to any preceding claim, wherein the mixture of nucleic acid and protein is contacted with the cation exchanger in a low molarity buffer.

7. A method according to Claim 6, wherein the buffer has a pH in the range 3.5-6.5.

8. A method according to any preceding claim, which is carried out automatically.

9. A method according to Claim 1 far the preparation of DNA for use as a target for DNA probe analysis.

10. Use of a cation exchanger in a method for the separation of nucleic acid from protein.

11. Use of a cation exchanger in a method according to any one of Claims 1-8.

1 2 3 4 5 6 7 8 9 10 11 12 $C_1$ $C_2$

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | CHEMICAL ABSTRACTS, vol. 109, no. 25, 19th December 1988, page 435, abstract no. 226275z, Columbus, Ohio, US; & SU-A-1 373 709 (INSTITUTE OF MEDICAL GENETICS) 15-02-1988 * The whole abstract * | 1-11 | C 12 P 19/34 C 12 N 15/10 |
| X | WO-A-8 404 309 (PHARMACIA) * The whole document * | 1-11 | |
| X | CHEMICAL ABSTRACTS, vol. 98, no. 7, 14th February 1983, page 353, abstract no. 50018f, Columbus, Ohio, US; & CS-A-199 123 (J. SIMUTH et al.) 30-09-1982 * The whole abstract * | 1-11 | |
| X | CHEMICAL ABSTRACTS, vol. 97, no. 17, 25th October 1982, page 548, abstract no. 143279m, Columbus, Ohio, US; E.S. STRASHNENKO et al.: "Use of ion-exchange for purifying protein hydrolyzates", & KONSERVN. OVOSHCHESUSH. PROM-ST. 1982, (6), 20-1 * The whole abstract * | 1-11 | **TECHNICAL FIELDS SEARCHED (Int. Cl.5)** C 12 P C 12 N |
| A | PATENT ABSTRACTS OF JAPAN, vol. 10, no. 131 (C-346)[2188], 15th May 1986; & JP-A-60 256 392 (KYOWA HAKKO KOGYO) 18-12-1985 | | |

The present search report has been drawn up for all claims

| Place of search | Date of completion of the search | Examiner |
|---|---|---|
| THE HAGUE | 29-01-1990 | PULAZZINI A.F.R. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)